# EUROPÄISCHE PATENTANMELDUNG

(11) **EP 4 245 143 A1**
(43) Veröffentlichungstag der Anmeldung: **20.09.2023**
(21) Anmeldenummer: 23152855.5
(22) Anmeldetag: 23.01.2023
(51) Int. Cl.: A01N 63/20, A01P 3/00

(54) **BIOLOGISCHES MITTEL ZUR VORBEUGUNG UND BEHANDLUNG VON BLATTKRANKHEITEN BEI PFLANZEN, WELCHE DURCH BIOTROPHE ODER HEMIBIOTROPHE PILZE AUSGELÖST WERDEN**

(30) Priorität: 14.03.2022 DE 102022105868
(71) Anmelder: Abitep GmbH, 12489 Berlin (DE)
(72) Erfinder: Beifort, Paul, 12487 Berlin (DE)
(74) Vertreter: Gulde & Partner

(57) **Zusammenfassung**

Die vorliegende Erfindung betrifft die Bereitstellung und Verwendung eines Mittels zur Vorbeugung und Behandlung von durch biotroph und hemibiotroph lebenden pilzlichen Erregern hervorgerufenen Blattkrankheiten bei Pflanzen, wobei das Mittel das Bakterium *Kosakonia radicincitans* enthält.

## Beschreibung

### Gegenstand der Erfindung:

Die Erfindung betrifft ein umweltverträgliches, wirksames, biologisches Mittel auf der Grundlage von nützlichen endophytischen Bakterien der Art *Kosakonia radicincitans* zum Schutz von Kulturpflanzen gegen pilzliche Blattkrankheiten, welche durch biotrophe oder hemibiotrophe Pilze ausgelöst werden. Dazu zählen die Erreger von Rostkrankheiten, z.B. Sojabohnenrost (*Phakopsora pachyrhizi*), Getreiderost (*Puccina sp*.), Gemüserost (*Puccina sp*.), Blattfleckenkrankheiten, z.B. *Septoria sp.* und echte und falsche Mehltaupilze, z.B. *Peronospora sp.* oder *Plasmopara sp.*

Vertreter der Art *Kosakonia radicincitans* besitzen die Fähigkeit zur Wurzel- und endophytischen Besiedelung von Kulturpflanzen. Gegenstand der Erfindung ist ein biologisches Mittel welches als Saatgutbehandlung und/oder Blattbehandlung zum Schutz gegen Blattkrankheiten eingesetzt wird.

Anwendungsgebiete der Erfindung sind Pflanzenschutz, Landwirtschaft und Gartenbau.

### Einleitung:

Die biologische Kontrolle von durch phytopathogene Pilze verursachte Blattkrankheiten stellt eine dringende Notwendigkeit dar, um den zunehmenden Bedarf an ökologisch unbedenklich hergestellten landwirtschaftlichen Produkten zu decken. Trotz des gesellschaftlichen Wandels der letzten Jahre in Bezug auf die gesetzlichen Vorgaben für die Zulassung von Pflanzenschutzmitteln beruhen die heutigen Praktiken in der Landwirtschaft immer noch größtenteils auf dem Einsatz synthetischer Pestizide, der genetischen Resistenz der Wirtspflanze und einem abgestimmtem Management von Pflanze und Umwelt. Gerade die andauernde Anwendung synthetischer Mittel mit einfachem Wirkmechanismus lässt diese über die Zeit ihre Wirksamkeit einbüßen, schafft Resistenzen bei den Erregern und wirkt sich häufig ungünstig auf die Umwelt aus. Besonders widerstandsfähig erweisen sich biotrophe und hemibiotrophe Pilze, deren Bekämpfung aufgrund ihrer teilweise im Blattinneren stattfindenden Vermehrung schwierig und häufig nicht durch äußerlich aufgebrachte Pflanzenschutzmittel erreichbar ist.

Die pilzlichen Erreger von Blattkrankheiten sind Mitglieder verschiedenster Pilzgattungen. Ihnen ist gemeinsam, dass sie in der Lage sind die Blattoberfläche bzw. das Blattinnere zu besiedeln und dafür in das Blatt einzudringen. Eine weitere Übereinstimmung ist, dass die Infektion mit dem pilzlichen Erreger mit einer optischen Veränderung des Blattes im weiteren Vegetationsverlauf einhergeht.

Generell kann man die pilzlichen Erkrankungen des Blattes auf Basis der nach der Invasion des Wirtes gewählten Ernährungsstrategie des Pilzes unterscheiden. Während nekrotrophe Pilze (z.B. *Phytophthora infestans, Botrytis cinerea*) das Wirtsgewebe schnell abtöten und im Anschluss saprophytisch leben, infizieren biotrophe und hemibiotrophe Pilze die Wirtspflanze ohne diese oder Teile von ihr für eine gewisse Zeit abzutöten.

Die biotroph und hemibiotroph lebenden Pilzarten töten das Gewebe der Wirtspflanze nicht unmittelbar ab, sondern bilden an der Blattoberfläche einen Pilzrasen oder Sporenlager aus, die als weißliche, gelbliche oder bräunliche Veränderungen der Blattoberfläche zu beobachten sind. Zu den Vertretern der durch biotrophe oder hemibiothrophe Pilze ausgelösten Blattkrankheiten zählen die Blattfleckenkrankheiten, die Rostkrankheiten und die Erkrankungen, welche durch Mehltaupilze ausgelöst werden.

Zu den wirtschaftlich bedeutsamen Blattfleckenkrankheiten zählen *Helminthosporium spp.* an Mais oder *Septoria sp.* an Getreide und Gemüsepflanzen. Bei den Rostkrankheiten sind die Erreger der Gattung *Puccinia sp.* hervorzuheben, welche den Zwergrost an Gerste, den Schwarzrost an Weizen, Spargelrost, Porreerost und Rostkrankheiten an vielen weiteren Kulturpflanzenarten verursachen. Weiterhin hat eine Rosterkrankung der Sojabohne, die durch den Erreger *Phakopsora pachyrhizi* hervorgerufen wird eine enorme wirtschaftliche Bedeutung. Der sogenannte asiatische Sojabohnenrost (SBR; *Phakopsora pachyrhizi* Syd.) führt zu Ertragseinbußen bei Sojabohnen (*Glycine max* (L.) Merr.), insbesondere in Gebieten, in denen die Krankheit endemisch ist und es an wirksamen Bekämpfungsmaßnahmen mangelt. Ertragsverluste von bis zu 80 % in Versuchen in Asien, 60 % in Brasilien und Paraguay und bis zu 27 % in Versuchsfeldern und 60 % in einem kommerziellen Feld in den Vereinigten Staaten wurden berichtet. Die Bekämpfung von Sojabohnenrost erfolgt in erster Linie durch den rechtzeitigen Einsatz von Fungiziden. Fungizide, die aus Triazolen, Strobilurinen und deren Mischungen bestehen, erwiesen sich in Versuchsflächen und auf kommerziellen Sojafeldern in Regionen Brasiliens und der Vereinigten Staaten als die wirksamste Methode zur Bekämpfung von Sojabohnenrost. Trotz der Wirksamkeit von Fungiziden zur Bekämpfung von Sojabohnenrost kann der intensive Einsatz von Fungiziden, insbesondere von Fungiziden mit einer einzigen Wirkungsweise, zu pathogenresistenten Populationen führen, was die Bekämpfung in den darauffolgenden Erntesaisons erschwert. So wurde beispielsweise berichtet, dass einige Triazolfungizide bei der Bekämpfung von Sojabohnenrost auf Feldern unwirksam sind.

Biologischen Pflanzenschutzmitteln stellen im Rahmen des Integrierten Pflanzenschutzes eine umwelt-freundliche Alternative zu chemischen Pestiziden dar, da sie oft einen komplexen Mode-of-action besitzen, der einer schnellen Resistenzbildung des Erregers entgegensteht.

Mittlerweile sind weltweit viele verschiedene Mikroorganismenarten als Aktivsubstanz von Pflanzenschutzmitteln registriert, jedoch keines auf der Basis eines endophytischen Bakteriums der Art *Kosakonia radicincitans.*

### Ziel und Aufgabe der Erfindung

Die Erfindung hat das Ziel, ein umweltverträgliches, wirksames Mittel gegen phytopathogene Pilze, auf der Grundlage definierter biologischer Faktoren zu entwickeln.

Aufgabe der Erfindung ist es, ein Mittel bereitzustellen zur Vorbeugung und Behandlung von pilzlichen Blattkrankheiten von Kulturpflanzen, welche von biotrophen oder hemibiotrophen Pilzen ausgelöst werden, wie die Erreger der Blattfleckenkrankheiten, z.B. *Septoria sp.,* der Rostkrankheiten, z.B. Getreide- und Gemüserost (*Puccina sp*.), des Sojabohnenrosts (*Phakopsora pachyrhizi*), d*e*r Erkrankungen der Blätter durch echte oder falsche Mehltaupilze z.B. *Peronospora sp.* oder *Plasmopara sp..*

### Beschreibung der Erfindung

Die erfindungsgemäße Lösung der Aufgabe ist in den Ansprüchen bestimmt.

Insbesondere betrifft die vorliegende Erfindung die Bereitstellung und Verwendung eines Mittels zur Vorbeugung und Behandlung von durch biotroph und hemibiotroph lebenden pilzlichen Erregern hervorgerufenen Blattkrankheiten bei Pflanzen, wobei das Mittel das Bakterium *Kosakonia radicincitans* enthält.

Kern der Erfindung ist der Einsatz des endophytischen Bakteriums der Art *Kosakonia radicincitans* als Saatgut- und Blattbehandlungsmittel zur Vorbeugung und Bekämpfung pilzlicher Blattkrankheiten ausgelöst durch biotrophe und hemibiothrophe Pilzarten, insbesondere Erreger der Blattfleckenkrankheiten, z.B. *Septoria sp.,* der Rostkrankheiten, z.B. Getreide- und Gemüserost (*Puccina sp.*), des Sojabohnenrosts (*Phakopsora pachyrhizi*), der Erkrankungen der Blätter durch echte oder falsche Mehltaupilze z.B. *Peronospora sp.* oder *Plasmopara sp.* an Blättern von Kulturpflanzen.

Es wurde überraschend gefunden, dass eine Behandlung des Saatguts bzw. der Blätter mit einem Mittel enthaltend das Bakterium *Kosakonia radicincitans,* mit der Hinterlegungsnummer DSM 16656 des Leibniz Instituts DSMZ, eine vorbeugende Wirkung gegen die Infektion mit biotrophen phytopathogenen Pilzen wie z.B. dem Sojabohnenrost besitzt.

Die vorliegende Erfindung bezieht sich auf ein Mittel zur Vorbeugung und Behandlung von durch biotroph und hemibiotroph lebenden pilzlichen Erregern hervorgerufenen Blattkrankheiten bei Pflanzen, wobei das Mittel das Bakterium *Kosakonia radicincitans* enthält.

Das erfindungsgemäße Mittel enthält als aktive Substanz das Bakterium *Kosakonia radicincitans.* Bevorzugt umfasst oder besteht das Bakterium *Kosakonia radicincitans* aus dem Stamm mit der Hinterlegungsnummer DSM16656. Das Bakterium *Kosakonia radicincitans* kann sowohl in flüssiger Kultur als auch in Festkultur kultiviert werden. Verfahren zur Kultur des Bakteriums *Kosakonia radicincitans* sind dem Fachmann bekannt.

Das erfindungsgemäße Mittel kann das Bakterium *Kosakonia radicincitans* in jeder beliebigen Form enthalten, bevorzugt enthält das Mittel das Bakterium *Kosakonia radicincitans* in einer Form, die Lagerstabil ist und eine Reproduktion bzw. (Re-)Kultivierung des Bakteriums erlaubt. Insbesondere kann das Mittel das Bakterium *Kosakonia radicincitans* in Form seiner Zellen und/oder in Form von Bestandteilen seiner Flüssig- oder Festkultur enthalten. Durch die Verwendung von lebenden Bakterienzellen kann eine Langzeitwirkung erzielt werden, die in der Fähigkeit des Bakteriums zur Besiedlung des Pflanzeninneren begründet liegt. Die Bakterienzellen findet man in großer Menge in der Flüssig- oder Festkultur von *Kosakonia radicincitans,* was eine einfache und kostengünstige Produktion des erfindungsgemäßen Mittels erlaubt.

Das erfindungsgemäße Mittel kann als Trockenpräparat oder als flüssiges Präparat formuliert sein. Das Mittel selbst kann also als Feststoff vorliegen, um ggf. später in Lösung genommen zu werden für die Verwendung des Mittels zur Behandlung von Samen/Saatgut oder Teilen von Pflanzen. Alternativ kann das Mittel auch in flüssiger Form vorliegen, wobei das Mittel das Bakterium *Kosakonia radicincitans* in einer Konzentration enthalten kann, die geeignet ist das Mittel für den Einsatz zur Vorbeugung und Behandlung von durch biotroph und hemibiotroph lebenden pilzlichen Erregern hervorgerufenen Blattkrankheiten bei Pflanzen. Das Mittel kann das Bakterium *Kosakonia radicincitans* auch in einer höheren Konzentration enthalten. In diesem Fall wird das Mittel gleichsam als Konzentrat bereitgestellt, welches vor der gewünschten Verwendung auf die beabsichtigte Konzentration verdünnt wird.

Zusätzlich kann das erfindungsgemäße Mittel noch weitere aktive Substanzen und/oder Hilfsstoffe aufweisen.

Als Hilfsstoffe können beispielsweise Stoffe zum Einsatz kommen, die die Formulierung des Mittels unterstützen, die Lagerstabilität erhöhen oder die Handhabung erleichtern. So kann das erfindungsgemäße Mittel beispielsweise einen oder mehrere Hilfsstoffe enthalten, die bevorzugt Zucker, Magermilchpulver, Glycerol, CaO, MgO, SiOz oder eine Kombination von mehreren der genannten Hilfsstoffe umfassen.

Als zusätzliche aktive Substanz kann das Mittel weitere chemische Verbindungen oder biologische Stoffe, wie z.B. weitere Mikroorganismen enthalten. In einer bevorzugten Ausführungsform enthält das erfindungsgemäße Mittel weitere Mikroorganismen wie beispielsweise zusätzliche Bakterien in Form von Sporen und/oder in Form von Bestandteilen der jeweiligen Flüssig- oder Festkultur. Besonders bevorzugt enthält das erfindungsgemäße Mittel zusätzlich Bakterien der Art *Bacillus velezensis, Bacillus atrophaeus, Bacillus pumilus, Bacillus simplex, Bacillus licheniformis* und/oder *Trichoderma sp..*

Die vorliegende Erfindung betrifft auch die Verwendung des erfindungsgemäßen Mittels zur Vorbeugung und Behandlung von durch biotroph und hemibiotroph lebenden pilzlichen Erregern hervorgerufenen Blattkrankheiten bei Pflanzen.

Für die Zwecke der vorliegenden Erfindung sind unter dem Begriff "Blattkrankheiten" solche Erkrankungen von Pflanzen zu verstehen, die durch biotroph und hemibiotroph lebende Pilze hervorgerufenen werden und optisch sichtbare Symptome oder Veränderungen an den Blättern zeigen, wie beispielsweise braune Verfärbungen, Läsionen und/oder Ansammlungen von Pilzsporen.

Bei den Blattkrankheiten, die durch das erfindungsgemäße Mittel behandelt werden können, handelt es sich bevorzugt um eine Blattfleckenkrankheit, eine Rostkrankheit und/oder eine Erkrankung der Blätter hervorgerufen durch echte oder falsche Mehltaupilze.

Den durch das erfindungsgemäße Mittel zu behandelnden Blattkrankheiten ist gemeinsam, dass diese durch biotroph und hemibiotroph lebende Pilze hervorgerufenen werden.

So werden beispielsweise Rostkrankheiten bei Pflanzen in der Regel durch Pilze der Ordnung Pucciniales (Rostpilze) hervorgerufen, bevorzugt durch Pilze der Gattungen Puccinia, Phakopsora, Hemileia, Aecidium und Uromyces, besonders bevorzugt der Gattungen Puccinia und Phakopsora, ganz besonders bevorzugt der Arten Phakopsora pachyrhizi, Puccinia sp. (insbesondere Puccinia graminis (Getreiderost), Puccinia sorghi (Maisrost), Puccinia striiformis (Gelbrost), Puccinia triticina (Braunrost des Weizens).

Eine Blattfleckenkrankheit wird in der Regel durch Pilze der Ordnung Pleosporales oder Capnodiales hervorgerufen, bevorzugt durch Pilze der Gattungen Septoria und Stagonospora, ganz besonders bevorzugt der Arten Septoria sp. (insbesondere Septoria tritici, Septoria lycopersici, Stagnospora nodorum).

Der echte Mehltau wird in der Regel durch Pilze der Ordnung Erysiphales hervorgerufen, bevorzugt durch Pilze der Gattung Blumeria, Erysiphe, Microsphaera, Oidium und Podosphaera, ganz besonders bevorzugt der Arten Blumeria graminis, Erysiphe sp. (insbesondere Erysiphe betae, Erysiphe communis, Erysiphe cruciferarum, Erysiphe graminis, Erysiphe necator).

Der falsche Mehltau kann beispielsweise durch folgende Pilze hervorgerufen werden: Pilze der Ordnung Peronosporales, bevorzugt durch Pilze der Gattungen Peronospora und Plasmopora, ganz besonders bevorzugt der Arten Peronospora sp. (insbesondere Peronospora tabacina) und Plasmopora sp. (insbesondere Plasmopora viticola).

Bevorzugt wird das erfindungsgemäße Mittel zur Vorbeugung und Behandlung von Blattkrankheiten bei Pflanzen verwendet, wobei die Blattkrankheit hervorgerufen wird durch einen pilzlichen Erreger ausgewählt aus der Ordnung Pucciniales (Rostpilze), Erysiphales, Peronosporales, Pleosporales und Capnodiales; bevorzugt aus den Gattungen Puccinia, Phakopsora, Hemileia, Aecidium, Uromyces, Septoria, Stagonospora, Blumeria, Erysiphe, Microsphaera, Oidium, Podosphaera, Peronospora und Plasmopora. Besonders bevorzugt wird das erfindungsgemäße Mittel zur Vorbeugung und Behandlung von Blattkrankheiten bei Pflanzen verwendet, wobei der pilzliche Erreger ausgewählt ist aus Phakopsora pachyrhizi, Puccinia sp. (insbesondere Puccinia graminis (Getreiderost), Puccinia sorghi (Maisrost), Puccinia striiformis (Gelbrost), Puccinia triticina (Braunrost des Weizens), Septoria sp. (insbesondere Septoria tritici, Septoria lycopersici, Stagnospora nodorum), Blumeria graminis, Erysiphe sp. (insbesondere Erysiphe betae, Erysiphe communis, Erysiphe cruciferarum, Erysiphe graminis, Erysiphe necator), Peronospora sp. (insbesondere Peronospora tabacina) und Plasmopora sp. (insbesondere Plasmopora viticola).

Bei der Verwendung des erfindungsgemäßen Mittels zur Vorbeugung und Behandlung von Blattkrankheiten bei Pflanzen wird das Mittel derart eingesetzt, dass das Saatgut oder ein Teil der zu behandelnden Pflanze mit dem Mittel in Kontakt gebracht wird. Dies kann durch jedes Verfahren geschehen, welches das Mittel derart mit dem Saatgut oder einem Teil der zu behandelnden Pflanze in Kontakt bringt, dass das Bakterium *Kosakonia radicincitans* auf einem Teil der Oberfläche des Saatguts oder des Pflanzenteils verbleibt oder von dem Saatgut bzw. dem behandelten Pflanzenteil wenigstens teilweise aufgenommen wird. Insbesondere kann das erfindungsgemäße Mittel zur Saatgutbehandlung, als Gießbehandlung, als Tröpfchenbewässerung, durch Tauchen, Zugabe zur Nährlösung in hydropischen Systemen, als Substratbeimischung oder als Blattspritzung angewendet werden.

Die vorliegende Erfindung bezieht sich auch auf ein Verfahren zur Vorbeugung und Behandlung von durch biotroph und hemibiotroph lebenden pilzlichen Erregern hervorgerufenen Blattkrankheiten bei Pflanzen, wobei ein erfindungsgemäßes Mittel verwendet wird. Das erfindungsgemäße Verfahren zeichnet sich insbesondere dadurch aus, dass das zu behandelnde Saatgut bzw. ein Bestandteil der zu behandelnden Pflanze mit dem Mittel in Kontakt gebracht wird. Dieses Inkontaktbringen kann auf unterschiedlichste Arten und Weisen erfolgen. So kann das Saatgut mit dem erfindungsgemäßen Mittel beschichtet werden bzw. mit einer flüssigen Lösung enthaltend das erfindungsgemäße Mittel benetzt oder darin untergetaucht werden. Die zu behandelnde Pflanze kann beispielsweise mit einer flüssigen Lösung enthaltend das erfindungsgemäße Mittel gegossen werden oder es können oberirdische Teile der Pflanze wie beispielsweise Blätter und/oder Stängel mit einer solchen Lösung besprüht oder bespritzt werden.

Die vorliegende Erfindung bezieht sich auch auf Saatgut, welches mit einem erfindungsgemäßen Mittel behandelt wurde. So kann das erfindungsgemäße Saatgut beispielsweise mit einer Lösung enthaltend das erfindungsgemäße Mittel in Kontakt gebracht worden sein (z.B. mittels Tauchen, Besprühen oder Beschichten). Bevorzugt weist das Saatgut auf mindestens einem Teil seiner Außenoberfläche das erfindungsgemäße Mittel auf. Als Saatgut im Sinne der vorliegenden Erfindung werden
ruhende, generative Fortpflanzungsorgane wie Samen, Früchte, Scheinfrüchte, Fruchtstände oder Teile davon bezeichnet. Das Saatgut enthält die vollständige, durch Befruchtung entstandene Keimanlage der betreffenden Pflanze.

Schließlich bezieht sich die vorliegende Erfindung auch auf eine Pflanze, wobei Teile der Pflanze mit einem erfindungsgemäßen Mittel in Kontakt gebracht wurden. Bevorzugt weist die Pflanze auf mindestens einem Teil ihrer Außenoberfläche das erfindungsgemäße Mittel gemäß auf, besonders bevorzugt weist die Pflanze auf mindestens einem Teil der Außenoberfläche ihrer Blätter das erfindungsgemäße Mittel auf.

### Figuren:

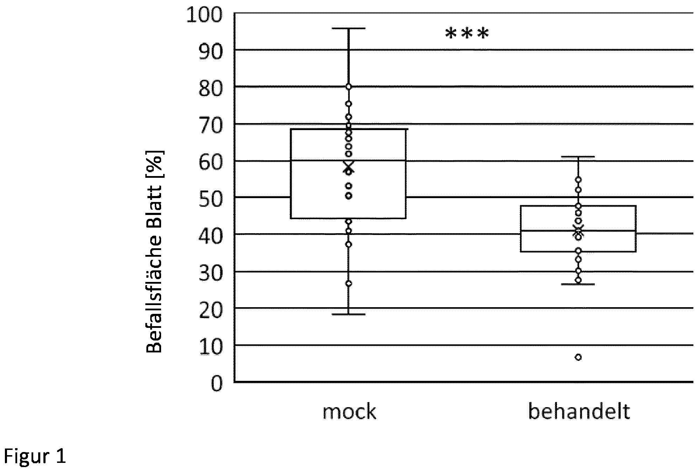

- Figur 1:: Wirksamkeitstest von Kosakonia radicincitans als Saatgutbehandlung gegen Phakopsora-Infektion des Primärblattes von Glycine max. Reduktion der Befallsfläche des Blattes (Rostpusteln, Läsionen und Chlorosen) nach Saatgutbehandlung mit Kosakonia radicincitans. Die Kontrolle (mock) zeigt mit 60% befallener Blattfläche einen signifikant höheren Befall, als die Blätter der am Saatgut mit Kosakonia radicincitans behandelten Sojapflanzen.

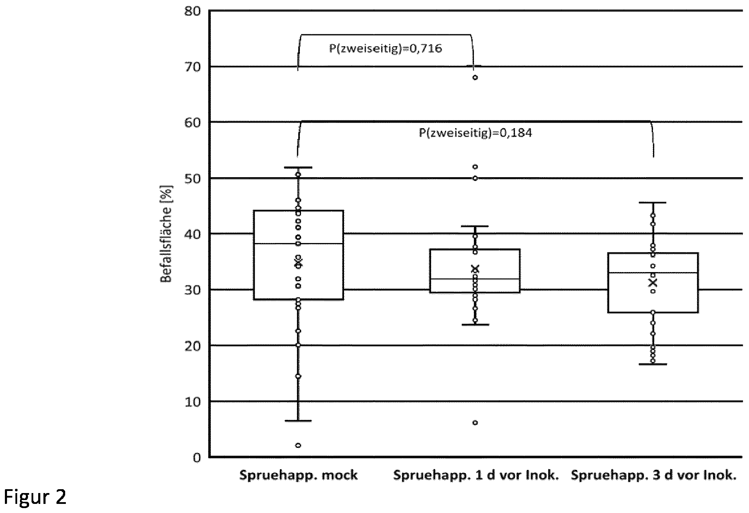

- Figur 2:: Wirksamkeitstest von Kosakonia radicincitans als Blattapplikation gegen Phakopsora-Infektion des Primärblattes von Glycine max. als Blattspritzung ein bzw. 3 Tage vor der künstlichen Infektion des Blatts. Die Kontrolle (mock) zeigt mit 38% befallener Blattfläche tendenziell einen höheren Befall, als die Blätter der als Blattspritzung mit Kosakonia radicincitans behandelten Sojapflanzen.

In den folgenden Ausführungsbeispielen wird die Erfindung näher erläutert.

### Ausführungsbeispiele:

### Wirksamkeit gegen Sojabohnenrost:

Zur Testung der Wirksamkeit gegen Sojabohnenrost wurde der Stamm *Kosakonia radicincitans* DSM16656 in 150mL SNB-Medium für 8h bei 200rpm inkubiert und durch Zentrifugation geerntet. Der so erhaltene Bioschlamm wurde mit 10mL steriler 0,9%iger NaCL-Lösung rückgelöst und das Sojabohnensaatgut (*Glycine max* var. Viola) darin für 1min bei Raumtemperatur kräftig geschwenkt. Das Saatgut wurde anschließend für 15 min im Luftstrom bei Raumtemperatur rückgetrocknet und danach für 3 Tage auf feuchtem 3MM Whatman-Papier zur Keimung ausgelegt. Ausgekeimte Sämlinge wurden am dritten Tag in Töpfe mit Fruhsdorfer Erde ausgepflanzt und ins Gewächshaus gebracht. 9 Tage nach dem Auspflanzen wurde die Inokulation der Primärblätter mit dem Pathogen *Phakopsora pachyrhizi* durchgeführt. Die Bonitur auf Läsionen erfolgte 12 Tage nach der Infektion der Blätter. Für die Blattapplikation wurden pro Pflanze 10E+08 lebende *Kosakonia radicincitans* Zellen mittels Blattspritzung einen bzw. drei Tage vor der Infektion des Primärblatts mit *Phakopsora pachyrhizi* aufgebracht.

Die Saatgutapplikation von *Kosakonia radicincitans* auf Sojabohnen zeigte eine signifikante Wirkung gegen Sojabohnenrost (Figur 1). Die Primärinfektion mit *Phakopsora pachyrhizi* um 12 % bei unnatürlich hohem Befallsdruck unter Laborbedingungen verringert werden. Dabei zeigte sich eine signifikante Reduktion der Rostpusteln, der Läsionen und der Chlorosen auf der Oberfläche der infizierten Primärblätter.

Eine Sprühapplikation auf die Blätter zeigte unabhängig vom Applikationszeitpunkt, ein oder drei Tage vor Infektion der Blätter, zwar noch keine signifikante Wirkung (Figur 2) aber einen klaren Trend hin zur Senkung der Befallsfläche des Blattes (Rostpusteln, Läsionen und Chlorosen). Es ist erkennbar, dass die Behandlung drei Tage vor Inokulation des Pilzes tendenziell eine bessere Wirkung aufweist, als die Behandlung einen Tag vor der Inokulation.

Neben verbesserter Krankheitsresistenz der Sojabohnenpflanzen zeigen die Ergebnisse, dass bei Saatgutbehandlung ein ausreichender Schutzeffekt gegen die Infektion mit asiatischem Sojabohnenrost erzielt werden kann (Figur 1). Die Blattspritzung ein oder drei Tage vor der künstlichen Infektion zeigte einen Trend zur Krankheitsvorbeugung (Figur 2 Figur 2). Die Ergebnisse von Saatgutbehandlung und Blattbehandlung lassen darauf schließen, dass sich das Bakterium erst in der Pflanze etablieren muss, bevor es seine vorbeugende Wirkung entfalten kann. Deshalb wird davon ausgegangen, dass die endophytische Lebensweise des Bakteriums allgemein Schutz vor phytopathogenen Pilzen bietet, die eine biotrophen bzw. hemibiotrophe Lebensweise besitzen, da diese mit *Kosakonia radicincitans* um den dortigen Lebensraum im Inneren der Pflanze konkurrieren müssen und ihnen so die Etablierung in der Wirtspflanze erschwert wird.

## Patentansprüche

1. Mittel zur Vorbeugung und Behandlung von durch biotroph und hemibiotroph lebenden pilzlichen Erregern hervorgerufenen Blattkrankheiten bei Pflanzen, wobei das Mittel das Bakterium *Kosakonia radicincitans* enthält.

2. Mittel gemäß Anspruch 1, wobei die Blattkrankheiten ausgewählt sind aus Blattfleckenkrankheit, Rostkrankheit und Erkrankungen der Blätter durch echte oder falsche Mehltaupilze.

3. Mittel gemäß einem der vorhergehenden Ansprüche, wobei das Bakterium *Kosakonia radicincitans* den Stamm mit der Hinterlegungsnummer DSM16656 umfasst oder daraus besteht.

4. Mittel gemäß einem der vorhergehenden Ansprüche, wobei das Mittel das Bakterium *Kosakonia radicincitans* in Form von Bakterienzellen und/oder in Form von Bestandteilen seiner Flüssig- oder Festkultur enthält.

5. Mittel gemäß einem der vorhergehenden Ansprüche, wobei das Mittel einen oder mehrere Hilfsstoffe enthält, bevorzugt Zucker, Magermilchpulver, Glycerol, CaO, MgO, SiOz oder eine Kombination von mehreren der genannten Hilfsstoffe.

6. Mittel gemäß einem der vorhergehenden Ansprüche, wobei es als Trockenpräparat oder als flüssiges Präparat formuliert ist.

7. Mittel gemäß einem der vorhergehenden Ansprüche, wobei das Mittel zusätzlich *Bacillus velezensis, Bacillus atrophaeus, Bacillus pumilus, Bacillus simplex, Bacillus licheniformis* und/oder *Trichoderma sp.* enthält, bevorzugt enthält das Mittel *Bacillus velezensis, Bacillus atrophaeus, Bacillus pumilus, Bacillus simplex, Bacillus licheniformis* und/oder *Trichoderma sp.* in Form von Sporen und/oder in Form von Bestandteilen der jeweiligen Flüssig- oder Festkultur.

8. Verwendung eines Mittels gemäß einem der Ansprüche 1 bis 7 zur Vorbeugung und Behandlung von durch biotroph und hemibiotroph lebenden pilzlichen Erregern hervorgerufenen Blattkrankheiten bei Pflanzen.

9. Verwendung nach Anspruch 8, wobei die Blattkrankheiten ausgewählt sind aus Blattfleckenkrankheit, Rostkrankheit und Erkrankungen der Blätter durch echte oder falsche Mehltaupilze.

10. Verwendung nach einem der Ansprüche 8 bis 9, wobei die Blattkrankheit hervorgerufen wird durch einen pilzlichen Erreger ausgewählt aus der Ordnung Pucciniales (Rostpilze), Erysiphales, Peronosporales, Pleosporales und Capnodiales; bevorzugt aus den Gattungen Puccinia, Phakopsora, Hemileia, Aecidium, Uromyces, Septoria, Stagonospora, Blumeria, Erysiphe, Microsphaera, Oidium, Podosphaera, Peronospora und Plasmopora.

11. Verwendung nach Anspruch 10, wobei der pilzliche Erreger ausgewählt ist aus Phakopsora pachyrhizi, Puccinia sp. (insbesondere Puccinia graminis (Getreiderost), Puccinia sorghi (Maisrost), Puccinia striiformis (Gelbrost), Puccinia triticina (Braunrost des Weizens), Septoria sp. (insbesondere Septoria tritici, Septoria lycopersici, Stagnospora nodorum), Blumeria graminis, Erysiphe sp. (insbesondere Erysiphe betae, Erysiphe communis, Erysiphe cruciferarum, Erysiphe graminis, Erysiphe necator), Peronospora sp. (insbesondere Peronospora tabacina) und Plasmopora sp. (insbesondere Plasmopora viticola).

12. Verwendung nach einem der Ansprüche 8 bis 11, wobei das Mittel zur Saatgutbehandlung, als Gießbehandlung, als Tröpfchenbewässerung, durch Tauchen, Zugabe zur Nährlösung in hydropischen Systemen, als Substratbeimischung oder als Blattspritzung angewendet wird.

13. Verfahren zur Vorbeugung und Behandlung von durch biotroph und hemibiotroph lebenden pilzlichen Erregern hervorgerufenen Blattkrankheiten bei Pflanzen, wobei ein Mittel nach einem der Ansprüche 1 bis 7 gemäß einem der Ansprüche 8 bis 12 verwendet wird.

14. Verfahren nach Anspruch 13, wobei Saatgut oder Blätter einer Pflanze mit einem Mittel nach einem der Ansprüche 1 bis 7 in Kontakt gebracht wird.

15. Saatgut, **dadurch gekennzeichnet, dass** das Saatgut mit einem Mittel gemäß einem der Ansprüche 1 bis 7 behandelt wurde, bevorzugt weist das Saatgut auf mindestens einem Teil seiner Außenoberfläche ein Mittel gemäß einem der Ansprüche 1 bis 7 auf.

16. Pflanze, **dadurch gekennzeichnet, dass** das Teile der Pflanze mit einem Mittel gemäß einem der Ansprüche 1 bis 7 behandelt wurde, bevorzugt weist die Pflanze auf mindestens einem Teil ihrer Außenoberfläche ein Mittel gemäß einem der Ansprüche 1 bis 7 auf, besonders bevorzugt weist die Pflanze auf mindestens einem Teil der Außenoberfläche ihrer Blätter ein Mittel gemäß einem der Ansprüche 1 bis 7 auf.
